# EUROPEAN PATENT APPLICATION

(11) **EP 4 345 796 A1**
(43) Date of publication of application: **03.04.2024**
(21) Application number: 22198637.5
(22) Date of filing: 29.09.2022
(51) Int. Cl.: G09B 19/00

(54) **IMPROVED GOLF SWING DETECTOR**

(71) Applicant: deWiz AB, 211 12 Malmö (SE)
(72) Inventor: Möller, Johan, Lund (SE); Smeds, Henrik, Lund (SE)
(74) Representative: Hansson Thyresson AB

(57) **Abstract**

There is provided a wearable device for improving a user's golf swing, the wearable device comprising: a sensor unit configured to sense motion sensor data; a stimulator for providing stimuli to the user of the wearable device; a processor; a central housing for housing the sensor unit, the stimulator, and the processor; wherein the processor is configured to detect that the user is in a golf stance position in dependence on a determined relative direction of gravity being within a pre-defined direction range indicative of a golf stance; wherein, in response to the detection of a golf stance position, the processor is further configured to: determine a motion track of the wearable device and transmit a stimuli signal to the stimulator causing the stimulator to provide stimuli to the user at a time when a golf swing flaw is detected in the determined motion track.

## Description

### TECHNICAL FIELD

The present invention relates to a sports training device for a user configured to issue a stimulus in response to a sport motion deviation of the user.

### BACKGROUND

Athletes exercising different sports often try to improve their technique, i.e., they try to improve their movements to achieve e.g., better golf shots, faster swimming or running, longer discus or javelin throws, higher precision archery shots etc. This is particularly true for sports involving a complicated movement pattern, such as in e.g., golf. One way of improving the technique is to take help of a coach or a video equipment to help study the technique to point out flaws or deviations compared to some ideal movement pattern. However, these methods, however successful, may be further improved by shortening the time from the user executing the flaw to the moment the user becomes aware of the movement being, in fact, flawed.

Some attempts along these lines have been more or less successful. A particular problem that has been difficult to solve is how to minimize the time from the moment when the flawed movement is performed by the user to the moment the user becomes aware of that the movement was in fact a flaw. This time may be called the "feedback delay time". Ideally, such feedback delay time should be as short as possible, preferably so short that the user perceives the feedback as instantaneous, such that the feedback is directly and unambiguously associated with the erroneous motion. The ideal feedback may be denoted "real time feedback" because there is no noticeable delay. In practice, it has been shown to be very hard to achieve such real time feedback.

From the above it is understood that there is room for improvements and the invention aims to solve or at least mitigate the above and other problems.

### SUMMARY

One object of the present invention is to provide a system capable of providing an electrical or other stimuli that can be perceived by a subject, at a moment in time coinciding with a motion irregularity corresponding to a sports motion fault performed by the subject.

The invention is defined by the appended independent claims. Additional features and advantages of the concepts disclosed herein are set forth in the description which follows, and in part will be obvious from the description, or may be learned by practice of the described technologies. The features and advantages of the concepts may be realized and obtained by means of the instruments and combinations particularly pointed out in the appended claims. These and other features of the described technologies will become more fully apparent from the following description and appended claims, or may be learned by the practice of the disclosed concepts as set forth herein.

In a first aspect, there is provided a wearable device for improving a user's golf swing, the wearable device comprising: a sensor unit configured to sense motion sensor data relating to acceleration and rotation of the wearable device in three-dimensional space; a stimulator for providing stimuli to the user of the wearable device; a processor; a central housing for housing the sensor unit, the stimulator, and the processor; and an attachment member for attaching the wearable device to a forearm of the user; wherein the processor is configured to: determine, in dependence on the motion sensor data, a relative direction of gravity relative to an orientation of the wearable device; detect that the user is in a golf stance position in dependence on the determined relative direction of gravity being within a pre-defined direction range indicative of a golf stance; wherein, in response to the detection of a golf stance position, the processor is further configured to: determine a motion track of the wearable device in dependence on motion sensor data from the sensor unit; and transmit a stimuli signal to the stimulator causing the stimulator to provide stimuli to the user at a time when a golf swing flaw is detect-ed in the determined motion track.

Preferably, the detection of the golf stance position is made in dependence on the determined relative direction of gravity being within the pre-defined direction range for an amount of time longer than a threshold amount of time.

Preferably. the processor is configured to determine if the wearable device is stationary, and wherein the detection of the golf stance position is made in dependence on the wearable device being determined to be stationary.

Preferably, the processor is configured to adapt the predefined direction range in dependence on a plurality of previously tracked golf swings, preferably in dependence on three to ten previously tracked golf swings, more preferably in dependence on three to five previously tracked golf swings.

Preferably, the predefined direction range is adapted in dependence of the determined relative direction of gravity for each of the plurality of previously tracked golf swings.

Preferably, the predefined direction range is adapted in dependence on either:
an average and variation amount of the determined relative direction of gravity of each of the plurality of tracked golf swings; or a graphical outlining of the determined relative direction of gravity of each of the plurality of tracked golf swings.

Preferably, the processor is further configured to determine if a golf swing has been completed, and wherein the plurality of previously tracked golf swings are golf swings that have been determined to be completed.

Preferably, the wearable device further comprises an indicator for indicating an internal state of the wearable device or a state of the tracked motion, wherein the indicator is configured to indicate to the user in dependence on detection of a golf stance position.

Preferably, the attachment member is one of a strap for a bracelet, a sleeve, or a glove.

Preferably, an arm-facing surface of the central housing or the attachment member comprises, or is provided with, a friction enhancing material; an arm-facing surface of the central housing comprises a curved portion; or the attachment member comprises a tensioning means for tensioning the attachment member to the forearm of the user.

Preferably, the processor is further configured to: determine, in dependence on the motion sensor data, a relative direction of gravity relative to the orientation of the wearable device when the user is in a golf stance position; determine one or more of (a) an arm mount position of the wearable device, (b) a golf club used by the user, and (c) a golf club group used by the user, by comparing the determined relative direction of gravity to a plurality of sub-ranges of a pre-defined direction range, each sub-range being indicative of (a) an arm mount position of the wearable device, (b) a golf club used by the user, or (c) a golf club group used by the user.

In a second aspect there is provided a method of detecting a golf stance position of a user, the method comprising: determining, in dependence on motion sensor data from a wearable device worn on a forearm of the user, a relative direction of gravity relative to an orientation of the wearable device; and detecting that the user is in the golf stance position in dependence on the determined relative direction of gravity being within a pre-defined direction range indicative of a golf stance.

In a third aspect there is provided a method of improving a golf swing of a user, the method comprising: determining, in dependence on motion sensor data from a wearable device worn on a forearm of the user, a relative direction of gravity relative to an orientation of the wearable device; detecting that the user is in a golf stance position in dependence on the determined relative direction of gravity being within a pre-defined direction range indicative of a golf stance; determining, in response to the detection of the golf stance position, a motion track of the wearable device in dependence on motion sensor data from the wearable device; and transmitting a stimuli signal to a stimulator of the wearable device causing the stimulator to provide stimuli to the user at a time when a golf swing flaw is detected in the determined motion track.

In a fourth aspect, there is provided a wearable device for improving a user's golf swing, the wearable device comprising: a sensor unit configured to sense motion sensor data relating to acceleration and rotation of the wearable device in three-dimensional space; a stimulator for providing stimuli to the user of the wearable device; a processor; a communication unit for communicating with a remotely located de-vice; a central housing for housing the sensor unit, the stimulator, and the processor; and an attachment member for attaching the wearable device to a forearm of the user; wherein the processor is configured to: determine, in dependence on the motion sensor data, a relative direction of gravity relative to an orientation of the wearable device when the user is in a golf stance position; determine one or more of (a) an arm mount position of the wearable device, (b) a golf club used by the user, and (c) a golf club group used by the user, by comparing the determined relative direction of gravity to a plurality of sub-ranges of a pre-defined direction range, each sub-range being indicative of (a) an arm mount position of the wearable device, (b) a golf club used by the user, or (c) a golf club group used by the user; determine a motion track of the wearable device in dependence on motion sensor data from the sensor unit; and transmit a stimuli signal to the stimulator causing the stimulator to provide stimuli to the user at a time when a golf swing flaw is detected in the determined motion track.

A further scope of applicability of the present invention will become apparent from the detailed description given below. However, it should be understood that the detailed description and specific examples, while indicating preferred embodiments of the invention, are given by way of illustration only, since various changes and modifications within the scope of the invention will become apparent to those skilled in the art from this detailed description. Hence, it is to be understood that this invention is not limited to the particular component parts of the device described or steps of the methods described as such device and method may vary. It is also to be understood that the terminology used herein is for purpose of describing particular embodiments only, and is not intended to be limiting. It must be noted that, as used in the specification and the appended claim, the articles "a," "an," "the," and "said" are intended to mean that there are one or more of the elements unless the context clearly dictates otherwise. Thus, for example, reference to "a sensor" or "the sensor" may include several sensors, and the like. Furthermore, the word "comprising" does not exclude other elements or steps.

### BRIEF DESCRIPTION OF THE DRAWINGS

In order to best describe the manner in which the above-described embodiments are implemented, as well as define other advantages and features of the disclosure, a more particular description is provided below and is illustrated in the appended drawings. Understanding that these drawings depict only exemplary embodiments of the invention and are not therefore to be considered to be limiting in scope, the examples will be described and explained with additional specificity and detail through the use of the accompanying drawings in which:
- Fig. 1: shows a user performing a golf swing;
- Fig. 2: shows a block diagram of a device according to embodiments;
- Fig. 3: shows a schematic diagram of a device according to embodiments communicating with external devices;
- Fig. 4a: shows a wearable device according to embodiments;
- Fig. 4b: shows a wearable device according to embodiments;
- Fig. 5: shows a method of detecting a golf swing flaw according to embodiments;
- Fig. 6: shows a method of detecting a golf swing according to embodiments;
- Fig. 7a: shows a user in a golf stance position;
- Fig. 7b: shows a side-view of a user in a golf stance position;
- Fig. 7c: shows a front view of a user in a golf stance position;
- Fig. 7d: shows a forearm of a user in a golf stance position;
- Fig. 8: shows a method of detecting a golf stance position according to embodiments;
- Fig. 9: shows a method of correcting a golf swing flaw according to embodiments;
- Fig. 10: shows a method of detecting a golf stance position according to embodiments;
- Fig. 11a: shows a relative direction of gravity in a device-centric reference frame;
- Fig. 11b: shows a relative direction of gravity in a device-centric reference frame;
- Fig. 12: shows a method of adapting a predefined direction range according to embodiments;
- Fig. 13: shows a method of adapting a predefined direction range according to embodiments;
- Fig. 14: shows a predefined direction range according to embodiments; and
- Fig. 15: shows a predefined direction range according to embodiments.

Further, in the figures like reference characters designate like or corresponding parts throughout the several figures. The first digit in the reference character denotes the first figure in which the corresponding element or part appears.

### DETAILED DESCRIPTION

Various embodiments of the disclosed methods and arrangements are discussed in detail below. While specific implementations are discussed, it should be understood that this is done for illustration purposes only. A person skilled in the relevant art will recognize that other components, configurations, and steps may be used without parting from the spirit and scope of the disclosure.

Hereinafter, certain embodiments will be described more fully with reference to the accompanying drawings. It will be apparent to those skilled in the art that various modifications and variations can be made without departing from the inventive concept. Other embodiments will be apparent to those skilled in the art from consideration of the specification and practice disclosed herein. It is to be understood that elements and materials may be substituted for those illustrated and described herein, parts and processes may be reversed or omitted, certain features may be utilized independently, and embodiments or features of embodiments may be combined, all as would be apparent to on skilled in the art.

The embodiments herein are provided by way of example so that this disclosure will be thorough and complete, and will fully convey the scope of the inventive concept. It should be understood that the description and the drawings are not intended to limit the invention to the particular form disclosed, but to the contrary, the intention is to cover all modifications, equivalents and alternatives falling within the spirit and scope of the present invention as defined by the appended claims. If nothing else is stated, different embodiments may be combined with each other.

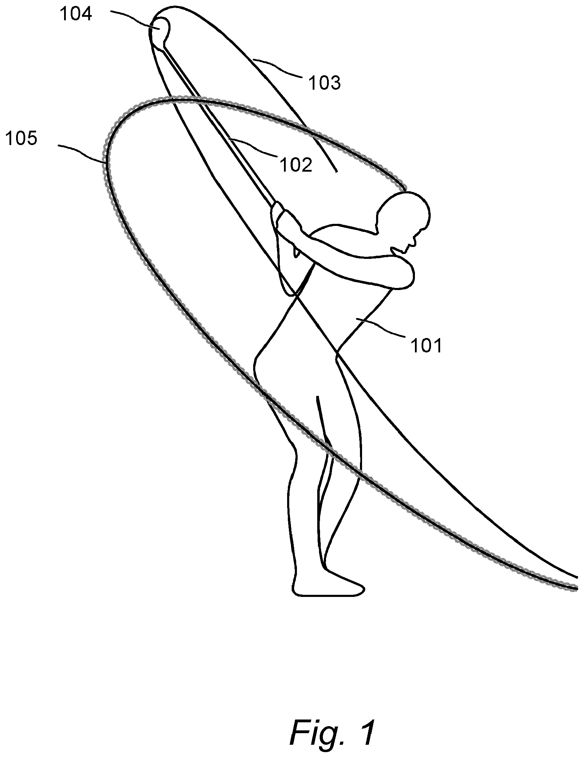

Fig. 1 shows a user 101 performing a golf swing. The user is holding a golf club 102. At the moment shown in Fig. 1, the user is partway through the golf swing. There is shown a motion track 103 that represents the motion of the head 104 of the golf club throughout the golf swing. There is also shown a reference motion track 105 which represents an ideal motion of a golf swing.

The actual golf swing performed by the user may, at times, deviate from the ideal reference motion. The inventors have realised that a person trying to improve a complex movement such as for example a golf swing, a baseball bat stroke, a pole vault, or a discus throw, by trying to achieve a motion track that is closer to the ideal reference motion, often have problems to correct the faulty portions of the motion, and to replace these faulty portions with more effective ones. Also, the person's coach, even though equipped with advanced training aids such as video recording equipment may find it difficult, and/or time consuming to help the person improving his or her motion.

Traditional video recording of an athlete's movement is of help, but need to be studied afterwards, when the movement is completed and the video device has been set to playback, and do not provide such fast feedback as may be desired. A video recording also has to be studied and processed using the cognitive function of the human brain. It may be an advantage to provide feedback instead, or also, using the more non-cognitive functions of the brain, such as the emotional functions or reflexes.

There is therefore provided a device for training aid, and a method of providing said training aid, that enables the user to learn new movement patterns also on a subconscious level that creates learning free from the conscious analytical mind. This in turn makes the new movement pattern sustainable under pressure. The device can elicit stimuli to the user at a time when the user's golf swing is flawed, e.g. when the golf swing differs from an optimal golf swing.

One problem involved in developing such a device is how to elicit stimuli at the precisely right time during execution of a golf swing. If the stimulus is provided in real time, at a time corresponding to when the golf swing deviates from the optimal golf swing, the user can subconsciously learn where the flaw occurs, and accordingly improve their golf swing. However, if stimulus is not elicited at the right time, it may not have a positive effect on the golf swing training of the user and may in worst cases have a negative impact user's training. The right moment in time may be defined as the moment in time when the position of the device in an ongoing motion track deviates from an ideal motion track in a way consistent with a golf swing flaw. In embodiments, there is considered to be a golf swing flaw if the ongoing motion track deviates from a reference motion track by more than a predefined threshold value. In embodiments, there is considered to be a golf swing flaw if the ongoing motion track deviates, by more than a threshold amount, from a reference plane (i.e. a swing plane) associated with an ideal (reference) golf swing.

Another problem is how to avoid eliciting stimuli during execution of a motion that is not a golf swing. The device thus has to be capable of differentiating a golf swing from a movement that is not a golf swing. Preferably, the device is capable of differentiating a golf swing from another movement at an early stage of the movement, so that stimulus can be elicited at an appropriate time. Indeed, it would not be helpful to determine whether the motion is a golf swing or not after the motion has been completed, because it would then be too late to elicit any useful stimuli.

In order to solve these problems, there is provided a device configured to determine, in real time, the current position of the device in relation to a predefined motion track, based on a determination of an initial position, a determination of an initial timestamp, and a determination of a reference direction.

The provided solution also solves related problems that have been identified by the inventor(s) during development of the device. Some of these problems are listed below:
- determine if the user is in a golf stance position (which can be used to assess whether the user is about to perform a golf swing);
- determine where on the body of the user the device is located, e.g. on which arm of the user the device is located, and/or which orientation the device has relative to the forearm of the user;
- determine which club or club group the user is using to perform the golf swing;
- indicating to the user that the device is ready to track the motion;
- displaying golf swing characteristics to a user.

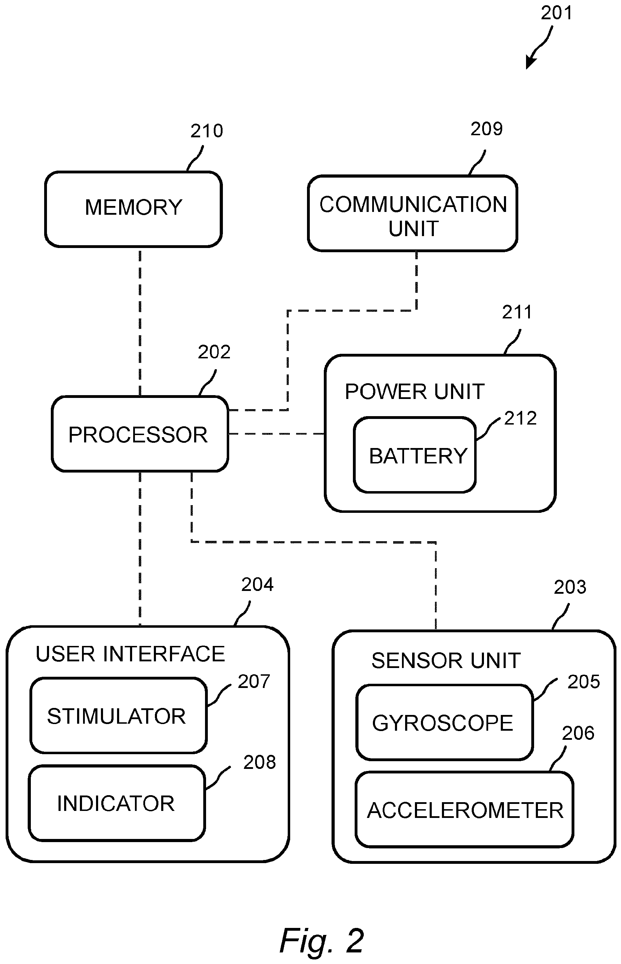

Fig. 2 shows a wearable device 201 according to embodiments which solves the above-identified problems. The wearable device 201 is worn by the user 101. The wearable device 201 may for example be worn on a wrist, a forearm, a shoulder, an arm, chest, or hip of the user, or any other location the movement of which represents the golf swing motion. In a preferred embodiment, the wearable device 201 is a worn on a forearm, and more preferably on the wrist or wrist portion of the forearm. The wearable device 201 is configured to be easily attachable to a body part of the user. The wearable device 201 may be in the form of, or form part of, a bracelet, a plaster, or in any other form that can be worn by the user. In a preferred embodiment, the wearable device 201 is in the form of a bracelet or a watch.

The wearable device 201 comprises a sensor unit 203, a processor 202, and a user interface 204.

The sensor unit 203 is able to sense motion sensor data, such as acceleration data, position data, velocity data, and/or gyroscopic data. The motion sensor data comprises data that enables an attitude and/or position of the wearable device 201 in three-dimensional space to be known or calculated. Attitude, in the context of embodiments, may be defined as an object's orientation (e.g. angular position) in space. The attitude may be represented by pitch, yaw and roll angles or, alternatively, by an attitude vector or axis, and a rotation angle around that vector or axis, i.e. axis-angle representation. The motion sensor data comprises data that enables changes in the attitude and/or position of the wearable device 201 to be known as the wearable device 201 moves during a user motion. The sensor unit 203 may comprise an accelerometer 206 and a gyroscope 205.

The sensor unit 203 is connected to the processor 202. The processor 202 is configured to process motion sensor data captured by the sensor unit 203. The processor 202 may be a single processor or a group of one or more processors.

The sensor unit 203 is configured to transmit captured motion sensor data to the processor 202, and the processor 202 is configured to track positions and/or attitude of the wearable device 201. The sensor unit 203 may preferably be a small integrated unit, such as a MEMS unit, providing accelerometer and gyroscopic data allowing the processor 202 to calculate relative position data of the sensor unit 203 without the need for external references. A commercially available sensor unit 203 is the semiconductor motion tracker device MPU-9250 from INVENSENSE, San Jose, California.

The user interface 204 of the wearable device 201 is an interface between a user and the internal components of the wearable device 201. The user interface 204 comprises a stimulator 207 for eliciting a stimulus to the user of the wearable device 201 in dependence on a tracked motion. The stimulus is perceptible to the user of the wearable device 201. The stimulator 207 is preferably configured to provide a discouraging stimulus. The stimulus may be a tactile stimulus, electrical stimulus, light stimulus, auditory stimulus, heat stimulus, or cold stimulus, or a combination thereof. Depending on the needs of the user the stimulus can be selected to maximize motor learning. Preferably the stimulus elicited by the stimulator 207 is an electric stimulus. Even more preferred, the stimulator 207 is configured to be able to elicit an electric stimulus of such magnitude that it is perceived as painful to most humans. The stimulator 207 is configured to be able to deliver such stimuli. The system may be configured such that the magnitude of the stimulus is adjustable. The stimulator 207 is configured to deliver the stimulus in real time, i.e., without noticeable delay, preferably with less delay than 50 milliseconds (ms), or more preferred less than 20 ms, or most preferred less than 10 ms.

The user interface 204 may additionally comprise an indicator 208 for indicating to the user a state of the wearable device 201 itself and/or of its tracked motion. The indicator 208 may be a visual indicator, a haptic indicator, an audio indicator, or a combination of the above. An audio-based indicator may be a sound signal which the user can audible hear. A haptic indicator may be based on vibration, such as a vibrating portion of the wearable device 201 which the user can feel through touch. A visual indicator may be provided by one or more lights, such as light emitting diodes (e.g. changing color, blinking), or by a user display located on the wearable device 201 which can indicate to the user a state of the wearable device 201 and/or tracked motion. A visual indicator may comprise light emitting diodes that change color as a state of the wearable device 201 and/or tracked motion changes. In an embodiment, a green light indicates a first state, an amber light indicates a second state, and a red light indicates a third stage. Although three states have been described here, it will be appreciated that further or less states may be denoted by specific colors, and that the colors green, amber, red are purely illustrative examples and other colors may thus be used. In another example, a sound signal emitted by the indicator 208 may change depending on the state of the wearable device 201 and/or tracked motion.

The wearable device 201 may comprise a communication unit 209 which enables communication between the wearable device 201 and one or more remotely located systems.

The wearable device 201 may comprise a memory 210 for storing motion sensor data. In alternative embodiments, a memory may be provided remotely from the wearable device 201, and such remotely located memory may be in communication with the wearable device 201 via the communication unit 209.

The wearable device 201 may comprise a power unit 211 for providing power to the wearable device 201. The power unit 211 may be in the form of a battery 212 or any other known component capable of providing power.

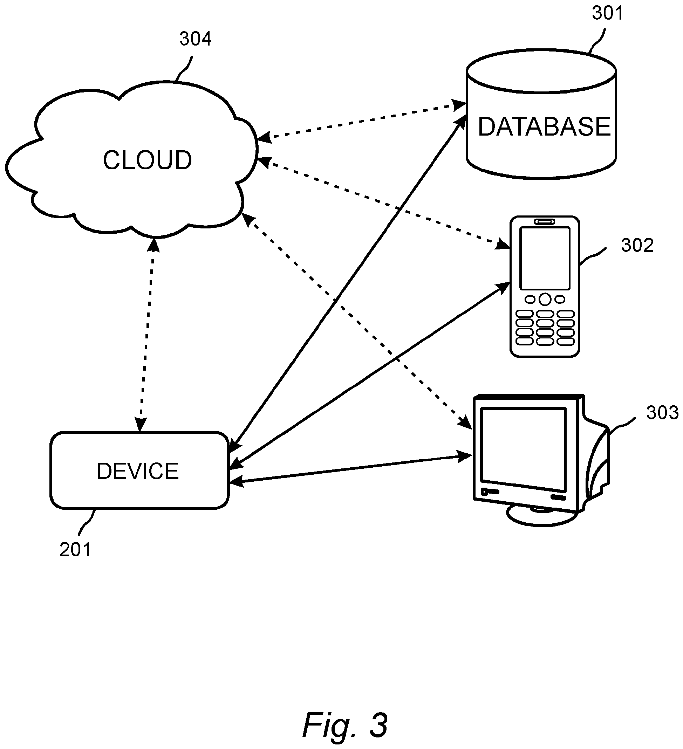

Fig. 3 shows a wearable device 201 communicating with one or more remotely located systems. The remotely located system(s) may be a computer 303, a smartphone 302, or the like. The remotely located system may alternatively be one or more databases 301. The remotely located system may comprise a memory unit that stores historic motion data provided by the wearable device 201 during previously tracked swing motions.

The communication unit 209 may communicate with the one or more remotely located systems via wireless communication means, such as the Internet, a cloud network, a Wireless Fidelity (Wi-Fi) network, a Wireless Local Area Network (WLAN), a Local Area Network (LAN), a telephone line (POTS), and/or a Metropolitan Area Network (MAN), a Bluetooth network or other network capable of transferring information between the wearable device 201 and the remotely located system. The communication between wearable device 201 and the remotely located system may be performed in accordance with various communication protocols, such as Transmission Control Protocol and Internet Protocol (TCP/IP), User Datagram Protocol (UDP), Hypertext Transfer Protocol (HTTP), File Transfer Protocol (FTP), ZigBee, EDGE, infrared (IR), IEEE 802.11, 802.16, cellular communication protocols, and/or Bluetooth (BT) communication protocols. The communication unit 209 may be connected to the remotely located system via one or more servers or cloud 304s, as indicated in Fig. 3.

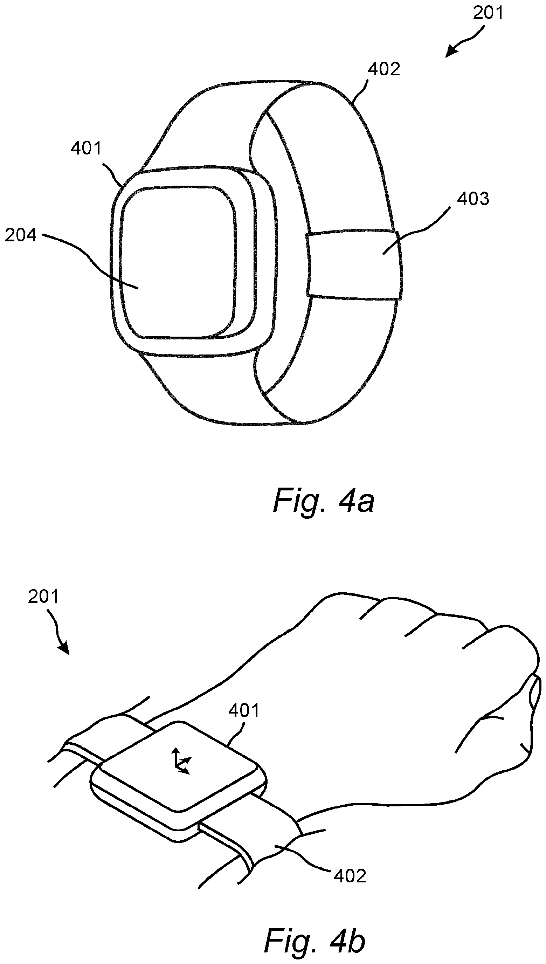

Figs. 4a and 4b show preferred embodiments in which the wearable device 201 is provided in the form of a bracelet. The wearable device 201 comprises a central housing 401 and an attachment member. The central housing 401 comprises the internal components of the wearable device 201, which were previously discussed in relation to Fig. 2, while the attachment member 402 allows the wearable device 201 to be attached to a wrist or forearm of a user. The attachment member 402 may for example be a strap 402 which allows the wearable device 201 to be strapped to the wrist or forearm of the user.

Embodiments include the wearable device 201 being provided in or on a glove. The wearable device 201 may be provided on or near the wrist of the glove. In this case, the attachment member may be the glove itself with the central housing attached thereto.

Embodiments also include the wearable device 201 being provided in or on a sleeve worn on the wrist or forearm of the user.

The embodiment shown in Fig. 4a shows a user interface 204 on a front surface of the central housing 401. There is provided a display and a button. The stimulator 207 of the user interface 204 may be placed on a back surface of the central housing 401 so as to be in contact with the user when the wearable device 201 is worn. There is also shown a tensioning means 403 which can tension the strap 402 so as to securely fit the wearable device 201 around the forearm of the user.

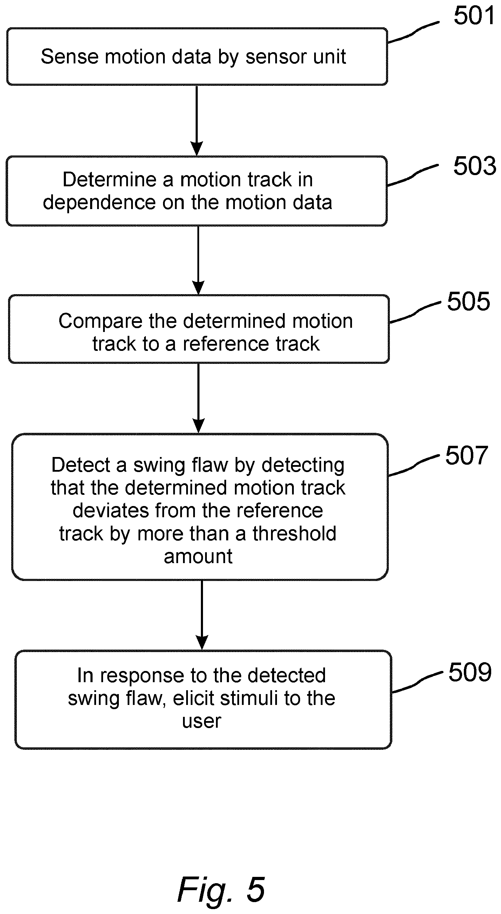

Fig. 5 shows a method of detecting a golf swing flaw.

In step 501, the sensor unit 203 is configured to sense motion data and transmit the sensed motion data to the processor 202.

In step 503, the processor 202 is configured to process the motion data from the sensor unit 203 in order to determine a motion track representing the motion of the wearable device 201 as the user moves through a golf swing.

In step 505, the processor 202 is configured to compare the position of the wearable device 201 along the determined motion track, at the specific point in time, to a reference track. The reference track may represent an ideal motion track that corresponds to a golf swing with no swing flaw.

In step 507, the processor 202 is configured to detect a swing flaw at a point in time when the determined motion track deviates from the reference track by more than a threshold amount. Upon detection of a swing flaw, the processor 202 is configured to transmit a stimuli signal to the stimulator 207.

In step 509, the stimulator 207 is configured to, in response to the stimuli signal from the processor 202, elicit stimuli to the user.

A golf swing flaw detection method according to embodiments is further described in European patent application EP16172927.2 which is incorporated by reference herein in its entirety.

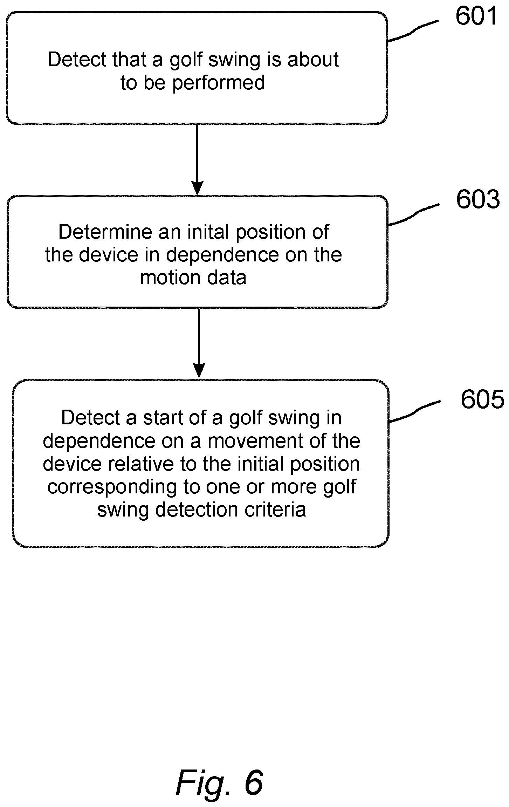

Fig. 6 shows a method of detecting a golf swing. It is advantageous to detect when a golf swing is performed. This allows the wearable device 201 only to elicit stimulus when a golf swing is performed, and not when another movement is made by the user. The golf swing detection may be seen as a pre-requisite for the method of detecting a golf swing flaw according to Fig. 5.

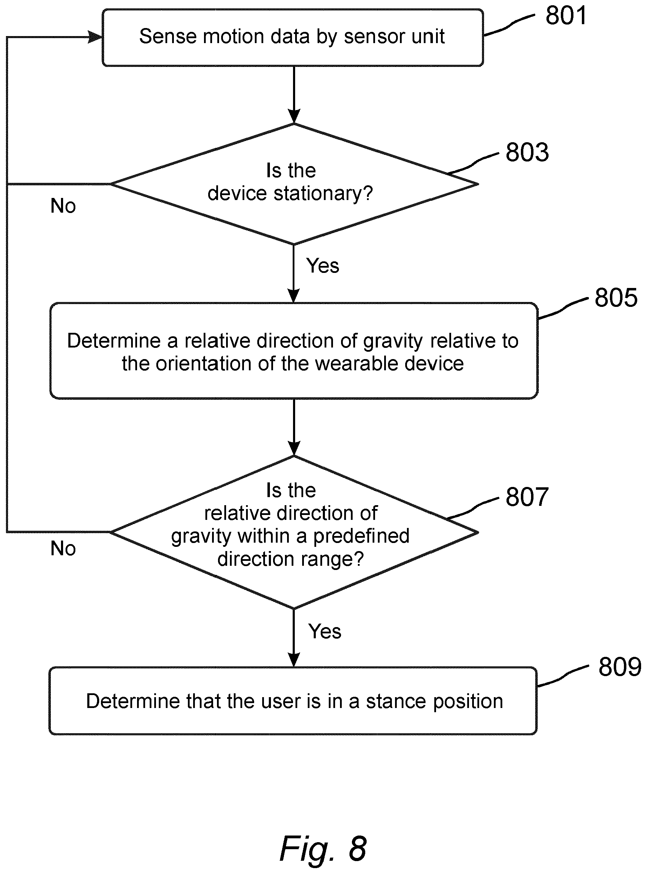

In the first step 601 of the golf swing detection method, the wearable device 201 is configured to detect that a golf swing is about to be performed. The inventors have realized that a good indicator of a golf swing being about to be performed is that the user is in a golf stance position. The golf stance position is the typical position a user is standing in, after having lined up the ball, and just before performing a golf swing. The method, and criteria, for detecting that a user is in a golf stance position will be explained in more detail in relation to Fig. 8.

In step 603, the processor 202 is configured to determine an initial position of the wearable device 201 using motion sensor data from the sensor unit 203. Subsequent motion of the wearable device 201 will be tracked compared to the initial position so as to remove the need for external reference points.

In step 605, the processor 202 is configured to detect a start of a golf swing in dependence on a movement of the wearable device 201, relative to the determined initial position, corresponding to one or more golf swing detection criteria. The golf swing detection criteria may be one or more of the following criteria:
1) the wearable device 201 moving in a vertical direction by more than a pre-determined height;
2) the wearable device 201 moving along an arc;
3) the wearable device 201 moving at a speed or angular speed higher than a pre-determined speed; and
4) the wearable device 201 experiencing a turn (i.e. a local minimum of speed and/or angular speed) within a predetermined time.

A method of detecting a start of a golf swing according to embodiments is described further in European patent application EP18180489.9 which is incorporated by reference herein in its entirety.

An imperative step of the golf swing detection method according to embodiments is to determine that a user is about to perform a golf swing. The inventors have therefore provided a method of detecting that a user is about to perform a golf swing by detecting that the user is in a golf stance position.

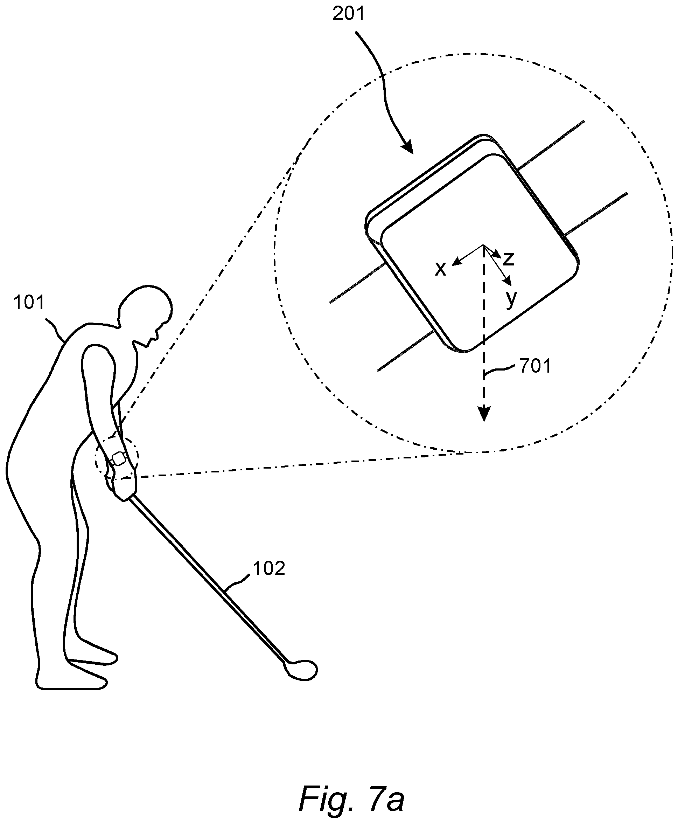

Figs. 7a to 7d show a user 101 about to perform a golf swing. The user 101 is in a stance position, which is the position a user typically takes before performing a golf swing.

The wearable device 201 according to embodiments can detect when the user is in a position which is a golf stance. This allows the wearable device 201 to determine when the user is likely to perform a golf swing, or more importantly, when the user is not likely to perform a golf swing. Accordingly, the wearable device 201 can more accurately determine when to track motion, and when to elicit stimuli. In a similar manner, the wearable device 201 can accordingly avoid tracking motion, and/or eliciting stimuli when the user has not initiated a golf swing (and been determined to be in a golf stance position).

Consider the device-centric right-hand coordinate system shown in Fig. 7a, where the axis parallel to the main surface of the wearable device 201 and along the strap 402 is denoted x, the axis parallel to the main surface of the wearable device 201 and orthogonal to the strap 402 direction is denoted y, and the axis orthogonal to the main surface of the wearable device 201 is denoted z. The direction of gravity 701 is also shown.

The inventors have realized that the orientation of the wearable device 201 can be used to determine if the user is in a golf stance position. Because the wearable device 201 is located on one of the user's forearms, and the user's forearms are typically in a specific manner in a golf stance position, the orientation of the wearable device 201 can be used to detect if the user is in a golf stance position.

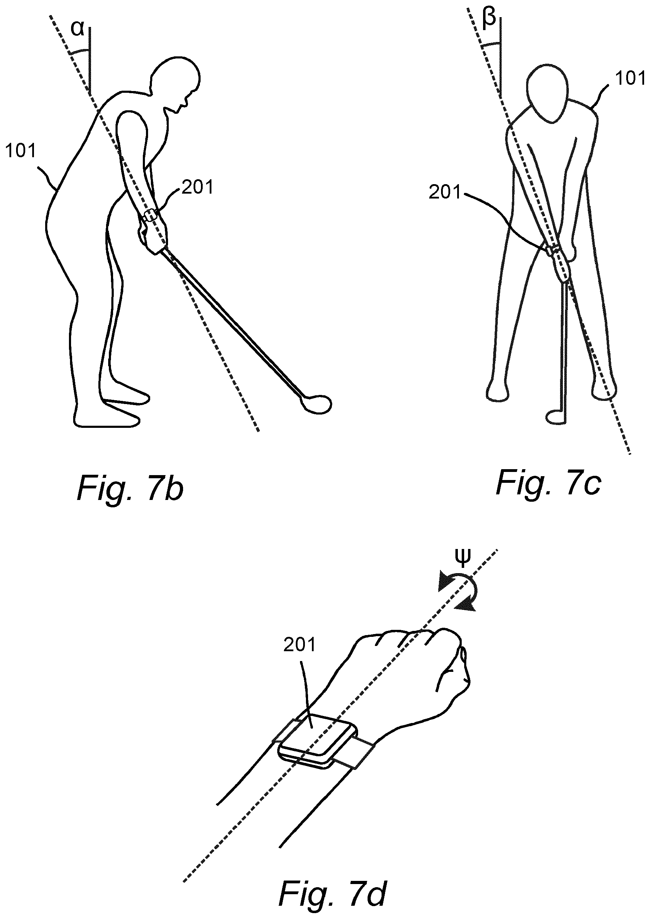

As shown in Fig. 7b, the orientation of the wearable device 201 is indicative of an angle α (alpha) of the forearm relative to the vertical (i.e. gravity) when considering the user in a sideview from behind the ball.

As shown in Fig. 7c, the orientation of the wearable device 201 is further indicative of an angle β (beta) of the forearm relative to the vertical (i.e. gravity) when considering the user in a front view.

Additionally, as shown in Fig. 7d, the orientation of the wearable device 201 is also indicative of a rotation ψ (psi) of the forearm around its own axis (for example a rotation of the wrist

The above-mentioned three indicators provided by the orientation of the wearable device 201, when considered together, gives a very good indication of whether the user is in a stance position or not.

The orientation of the wearable device 201 (when worn on the forearm) is thus a more accurate indicator of a golf stance position than the orientation of the golf club. The orientation of a golf club is only indicative of angles alpha and beta, because in a golf stance position the face of the golf club will always be rotated to face the ball. Furthermore, there may be several other occasions when the golf club is held at specific angles of alpha and beta corresponding to a golf stance position without a golf swing being about to be performed. For example, the user may accidentally hold the golf club at this angle, or the golf club may be leaning against a fence or the like. At these occasions a device worn on the forearm is superior in detection of a golf stance position because the wearable device 201 can additionally indicate the rotation of the forearm around its own axis. A device worn on a wrist portion of the forearm offers even more superior detection of a golf stance position.

These three measures, when considered together, gives a very good indication of whether the user is in a stance position or not.

The provided method and wearable device 201 also take into account, by detecting both the angles and rotation of the forearm, how a specific user is typically holding a golf club. The determination of whether a user's position is a stance position, can thus be more accurately tailored to specific users. For example, different users may typically hold the golf club with different grips leading to different wrist rotations.

Furthermore, the inventors have realized that for a wearable device 201 in the form of a bracelet comprising a strap member and a central housing 401, the orientation of the wearable device 201 closely resembles the orientation of the forearm on which it is worn. The strap of the wearable device 201, which is strapped around the forearm of the user, ensures that y-axis and x-axis are always substantially aligned along and perpendicular to the longitudinal direction of the forearm respectively. Furthermore, since the central housing 401 of the wearable device 201 is substantially flat, the central housing 401 of the wearable device 201 rests on the forearm of the user, such that the z-axis of the wearable device 201 always points away from the forearm in a specific direction (relative to the forearm).

Additionally, a rotational degree of freedom of the wearable device 201, compared to the forearm to which it is attached, may preferably be reduced, or more preferably removed. This may be achieved by one or more of:
an arm-facing surface of the central housing 401 and/or the strap 402 of the wearable device 201 may be made from, or provided with, a friction enhancing material which functions to increase the friction between the forearm and the device so as to reduce a rotation amount of the device during movement of the forearm;
an arm-facing surface of the housing portion may be provided with a curvature such that the arm-facing surface more closely conforms to the forearm, and/or a certain part of the forearm, of the user; and
the strap 402 being provided with a tensioning means 403 for tensioning the strap 402 to the forearm of the user. The tensioning means 403 may be a stretchable portion of the strap 402, a length-adjusting portion of the strap 402 or a combination of the above.

It can accordingly be ensured that the orientation of the wearable device 201 closely corresponds to the orientation of the forearm of the user to which it is attached.

The method of detecting that the user is in a golf stance position is provided in Fig. 8.

In step 801, the sensor unit 203 is configured to sense motion data. The motion data may be acceleration data only. The acceleration data comprises acceleration data in 3 orthogonal directions, for example in the x-, y-, and z-directions of the coordinate system in Fig. 7.

In step 803, the processor 202 is configured to determine if the wearable device 201 is stationary. A criterion for determining that the wearable device 201 is stationary may be that the sensed acceleration amount substantially corresponds to the gravity acceleration. A method of detecting that the wearable device 201 is stationary has been further explained in European patent application EP18180484.0 which is incorporated herein by reference in its entirety. If the wearable device 201 is stationary, then the wearable device 201 is configured to carry out step 805. If the wearable device 201 is not stationary, the wearable device 201 is configured to go back to step 801.

In step 805, the processor 202 is configured to determine the direction of gravity. The direction of gravity is determined in dependence on the acceleration data sensed by the sensor unit 203. Since the wearable device 201 is stationary, the acceleration data comprises substantially only a gravity component. The direction of gravity is determined relative to the orientation of the wearable device 201. For example, the acceleration data sensed by the sensor unit 203 may comprise a first amount of acceleration in an x-direction, a second amount of acceleration in a y-direction, and a third amount of acceleration in a z-direction. The first, second, and third amounts of gravity may be used to determine the direction of gravity relative to the wearable device 201. This may alternatively be seen as the direction of gravity being determined in a device-centric reference frame.

In step 807, the processor 202 is configured to compare the determined relative direction of gravity to a predefined direction range. This comparison is preferably performed in real-time. The inventors have realized that, for any specific user, the direction of gravity relative to the orientation of the wearable device 201 is within a limited range when the user is in a golf stance position. Thus, if the determined relative direction of gravity is within a predefined direction range, the wearable device 201 can determine that the user is likely to be in a stance position.

The predefined direction range may be defined in dependence on a typical golf stance position of a user. In particular, the predefined direction range may be defined in dependence on typical angles α (alpha) of the forearm relative to gravity in a side view from behind the user, typical angles β (beta) of the forearm relative to gravity in a front view of the user, and/or typical rotation amounts ψ (psi) of the forearm around its own axis (e.g. rotation of wrist relative to elbow), associated with a golf stance position.

If the determined relative direction of gravity is (instantaneously) within the predefined direction range, the processor 202, in step 809, is configured to determine that the user is in a stance position. If the determined relative direction of gravity is outside of the predefined direction range, the user is determined not to be in a stance position.

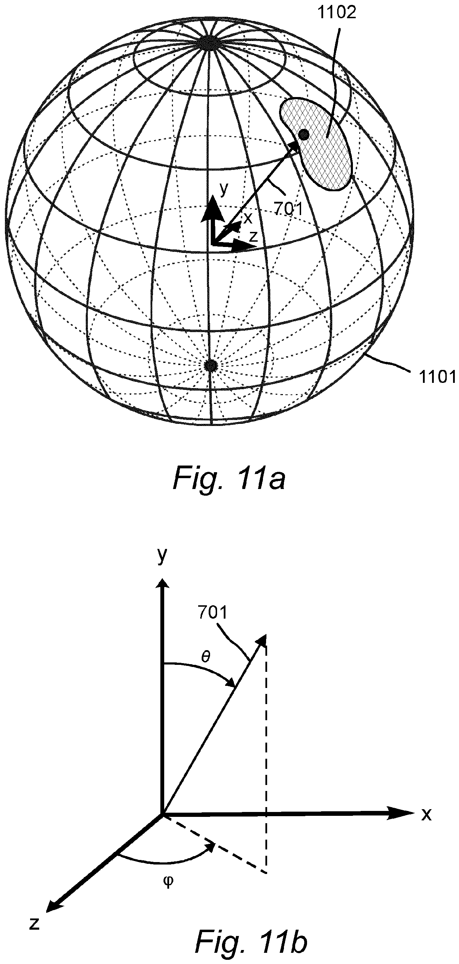

The pre-defined direction range will be further explained in relation to Figs. 11 to 15.

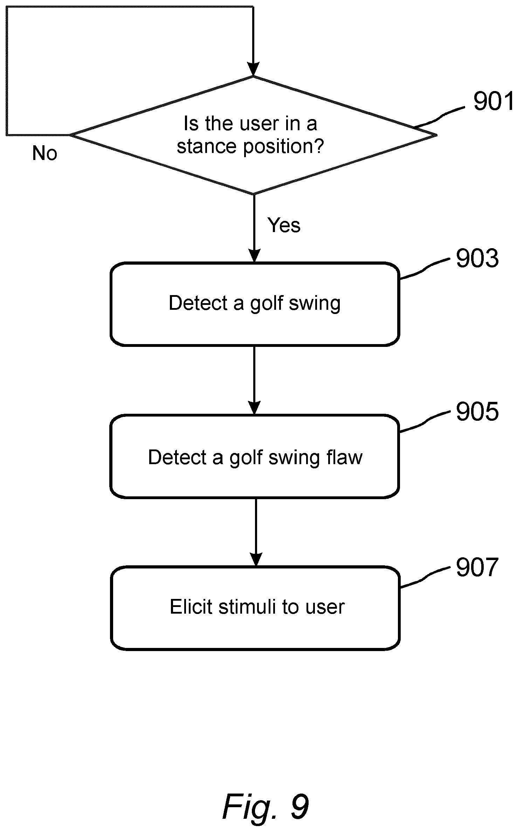

As shown in Fig. 9, the wearable device 201 may be configured to only track detailed motion and elicit stimuli after the user has been detected to be in a golf stance position. The wearable device 201 can therefore avoid tracking motion and/or eliciting stimulus for a movement that is not a golf swing. Examples of such movements include random movement by the user, warm-up motions performed by the user before starting the golf swing, or any other movement the user may make that is not a golf swing.

In step 901, the wearable device 201 detects if the user is in a golf stance position. In response to the user being detected to be in a golf stance position, the wearable device 201 may indicate to the user, via the indicator 208, that the device is ready to track a golf swing.

In step 903, in response to the user being detected to be in a golf stance position, the wearable device 201 is configured to track the motion of the wearable device 201 and detect a golf swing.

In step 905, the wearable device 201 is configured to detect a golf swing flaw.

In step 907, the wearable device 201 is configured to elicit stimuli to the user in response to the detected golf swing flaw.

The wearable device 201 may further be configured to keep one or more internal components (e.g. one or more sensors, stimulator 207, communication unit 209, user interface 204 or the like) inactive until the user has been determined to be in a stance position. Such internal components may be (highly) energy consuming. Thus, battery power can be saved by allowing the wearable device 201 to keeping them turned off when the user has not been detected to be in a stance position. In embodiments, the wearable device 201 can turn off one or more internal components upon completion of a golf swing. In embodiments, the wearable device 201 can turn on one or more internal components upon detection of the user being in a stance position.

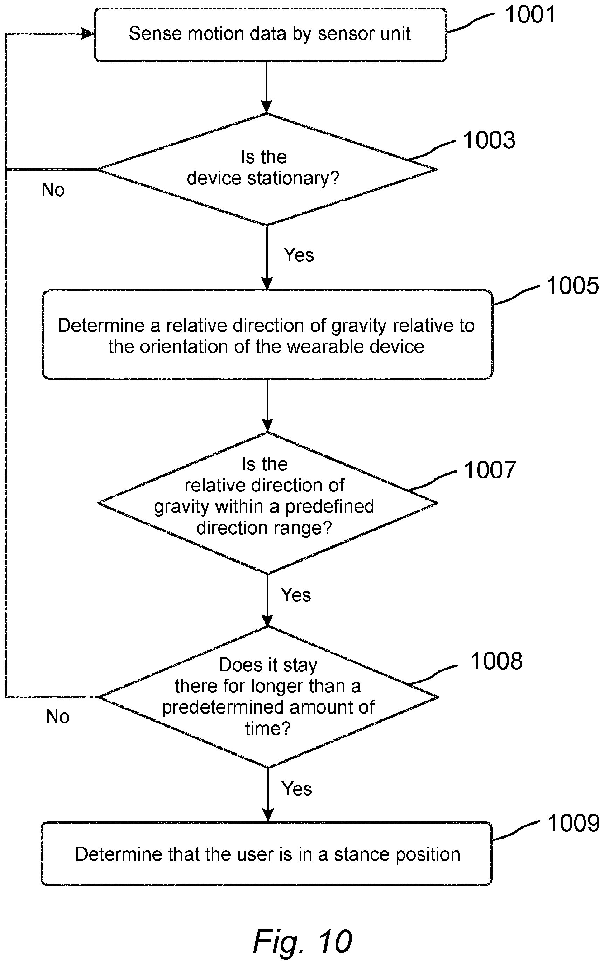

Fig. 10 shows a preferred method of determining when a user is in a stance position. The method is substantially the same as the method described in relation to Fig. 8, but the method comprises one more additional step.

Steps 1001, 1003, 1005, 1007, and 1009 respectively corresponds to steps 801, 803, 805, 807, and 809 of the previously described method.

An additional step 1008 is provided. In step 1008, the processor 202 is configured to determine if the relative direction of gravity remains within the predefined direction range for more than a predetermined amount length of time. This step increases the accuracy of the golf stance detection method, by excluding cases where the user is accidentally and briefly in a golf stance position. Typically, while the user is in the stance position, the user may aim the golf shot, visually focus on the ball, adjust their grip on the golf club or the like. It is therefore likely that the user will be still in the stance position for a certain amount of time. Thus, in embodiments, the wearable device 201 may determine that the user is in a stance position in dependence on the determined relative direction of gravity being within the certain direction range for a pre-determined amount of time. The pre-determined amount of time may be between 5 to 20 milliseconds, preferably between 7 to 15 milliseconds, or more preferably 9 to 11 milliseconds.

Fig. 11a shows a device-centric reference frame using the coordinate system introduced in Fig. 7. The direction of gravity 701 is shown in said device-centric reference frame. There is also shown an imaginary sphere 1101 centered around the origin of the coordinate system of the wearable device 201. It is to be understood that the coordinate system and the imaginary sphere are provided solely for illustrative purposes.

The predefined direction range for a golf stance position may be illustrated as an area 1102 on the surface of the imaginary sphere 1101. The wearable device 201 may thus determine that the user is in a golf stance position if the determined relative direction of gravity corresponds to a pre-defined area 1102 on the imaginary sphere. That is, if a direction vector 701 for the relative direction of gravity intersects with the imaginary sphere 1101 at a location within the pre-defined area 1102, then the user may be considered to be in a golf stance position.

Although the figure shows a device-centric reference frame, it will be appreciated that the same determination could be made in a global reference frame (where gravity is pointing downwards).

As shown in Fig. 11b, the relative direction of gravity may be represented, in the device-centric reference frame, as a unit vector 701 using polar coordinates theta *θ* and phi ϕ. The predefined direction range may be defined by a variation in the *θ*-amount and ϕ-amount from respective reference values *θ*₀ and ϕ₀. For example, the predefined direction range may be defined as *θ* = *θ*₀ ± Δ*θ*; ϕ = ϕ₀ ± Δϕ. It will be appreciated that embodiments are not limited to this specific definition of the predefined direction range, and that embodiments include other definitions defining a suitable shape on the surface of the imaginary sphere.

Although the predefined direction range has been described using polar coordinates *θ* and ϕ, it will be appreciated that the predefined direction range can be defined in any suitable way, and the predefined direction range can form any suitable shape on the surface of the imaginary sphere indicative of a golf stance position.

The predefined direction range may be dynamically adapted in dependence on previous golf swings performed by the user. In the example given above, the variation and/or the reference value of *θ* and/or ϕ may be adapted in dependence on previous golf swings. For example, the direction range may be reduced to correspond to a more limited range.

Initially, *θ*₀, ϕ₀, Δ*θ*, and Δϕ (or other parameters defining the predefined direction range) may be determined experimentally or empirically to correspond to a direction range which includes the relative direction of gravity for most (preferably substantially all, and more preferably all) golf players in their golf stance positions. The variations Δ*θ* and Δϕ may thus be relatively large, so as to define a relatively large, predefined area.

For example, experiments have shown that initial values of Δ*θ* and Δϕ between approximately 30 to 50 degrees are suitable for most golfers.

The predefined direction range may subsequently be adapted to correspond more closely to the golf stance of a specific user. For example, the parameters Δ*θ*, and Δϕ may be reduced after a number of completed golf swings.

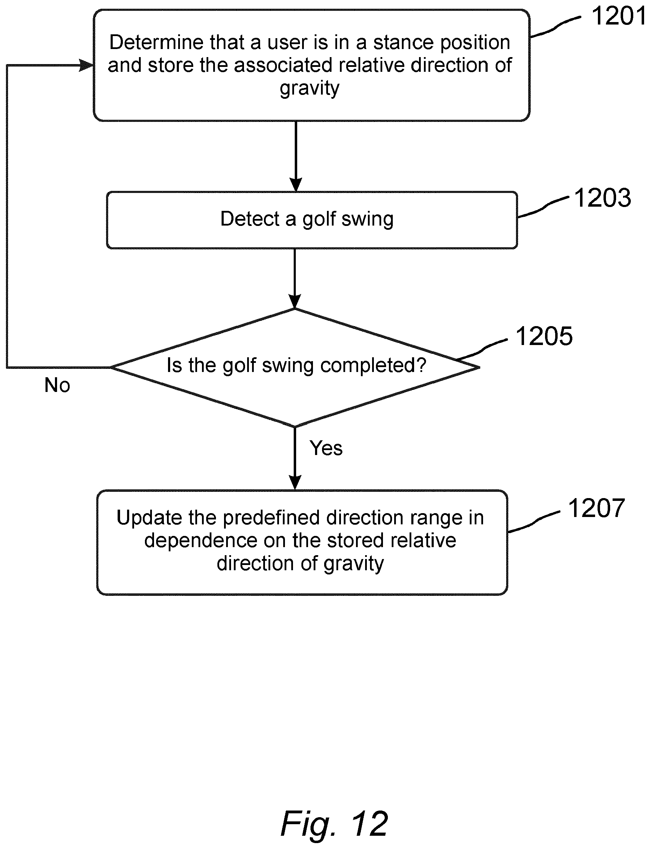

Fig. 12 shows a method of adapting the predefined direction range in dependence on previous golf swings performed by the user.

In step 1201, the wearable device 201 determines that a user is in a golf stance position according to the method of Fig. 8 or 10. The wearable device 201 stores the relative direction of gravity for that golf stance. The relative direction of gravity may be stored in an internal memory 210 of the wearable device 201, or by means of an external memory with which the wearable device 201 can communicate via the communication unit 209.

In step 1203, the wearable device 201 is configured to detect a golf swing according to the method previously described in relation to Fig. 6.

In step 1205, the wearable device 201 is configured to track the motion of the wearable device 201 and determine whether the golf swing has been completed, is ongoing, or has been discontinued. The wearable device 201 may be configured to determine that the golf swing is completed by detecting an impact with the golf ball within a predetermined amount of time from the start of the golf swing. For example, a sudden change in accelerometer data may be detected at a time of impact with the golf ball. The wearable device 201 may alternatively be configured to determine that the golf swing is completed by detecting that the wearable device 201 follows a motion path comprising at least portions of: a back swing, a turn, and a down swing.

The wearable device 201 may further determine that the golf swing has been discontinued if the golf swing has not been determined to be completed within a predetermined amount of time. If the golf swing is discontinued, the wearable device may be configured to go back to step 1201.

If the wearable device 201 has not determined that the golf swing is completed or discontinued, the wearable device 201 may be configured to assume that the golf swing is ongoing.

In step 1207, if the golf swing has been determined to be completed, the wearable de-vice 201 is configured to adapt the predefined direction range in dependence on the stored relative direction of gravity for the performed golf swing, and to store the adapted predefined direction range in memory. In one embodiment, the wearable device 201 is configured to adapt the predefined direction range irrespective of whether a completion of the golf swing has been detected.

Two different methods of performing the adaption of the predefined direction range will be explained here.

In a first method, the predefined direction range is adapted based on averaging the relative directions of gravity stored for a plurality of previous golf swings. The reference values *θ*₀ and ϕ₀ may be adapted to be the average of the respective angles associated with the relative directions of gravity for the plurality of golf swings. The variations Δ*θ* and Δϕ may be adapted to be defined in dependence on the maximum deviation amount from the average values of *θ* and ϕ of the relative directions of gravity for the plurality of golf swings. For example, the variations Δ*θ* and Δϕ may be adapted to be defined as a multiple of the maximum deviation amount, (e.g. x 1, x 1.2, x 1.5, x 2 etc.).

Alternatively, the variations of Δ*θ* and Δϕ may be set to predetermined values that are lower than their initial values. For example, the variations Δ*θ* and Δϕ may be reduced after a predetermined number of golf swings. As another example, Δ*θ* and Δϕ may be set, after a predetermined number of golf swings, to be approximately half of the initial values of Δ*θ* and Δϕ. Experiments have shown that suitable values of Δ*θ* and Δϕ, after at least three golf swings, are between 10 and 25 degrees.

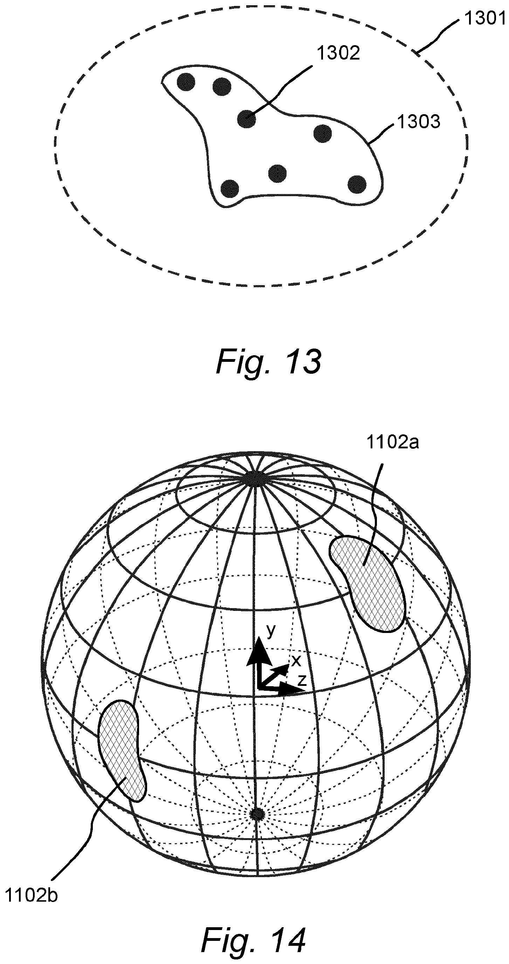

In a second method, the predefined direction range may be adapted by creating an outline around relative directions of gravity stored for a plurality of previous golf swings. Fig. 13 shows a 2D projection of an initial predefined direction range 1301 together with intersections 1302 between vectors representing the relative directions of gravity for a plurality of previous golf swings and the imaginary sphere of Fig. 7. In this instance, there is shown seven intersections 1302, representing the stored relative directions of gravity for the seven last performed golf swings respectively. There is also shown an adapted predefined direction range 1303, taking the seven previous golf swings into account. As can be seen, adapting the predefined direction range allows the predefined direction range to be user specific based on previous golf swings. The outlining of the intersections 1302 may be performed using any known method for outlining data points.

Although seven golf swings were taken into account in the previous example, the plurality of previous golf swings taken into account when adapting the predefined direction range may be any number that allows an accurate direction range to be obtained. The adaption process may be based on, for example, 2 golf swings, 3 golf swings, 5 golf swings, 10 golf swings, or more than 10 golf swings. Basing the adaption process on 3 golf swings has been found to be particularly advantageous since it allows the predefined direction range to be tailored to the specific user, while still being flexible enough to allow the user to change their stance position slightly without the wearable device 201 not recognizing the stance position.

In an embodiment, the number of golf swings taken into account may be increased over time as the user performs more and more golf swings. For example, when the user starts using the wearable device 201, 3 golf swings may be used, but after the user has performed more golf swings, the number of golf swings may increase, e.g. to 5 or 10. In an embodiment, all stored golf swings for a specific user is taken into account.

The predefined direction range may be reset at the start of every new practice session. This may be every time the wearable device 201 is restarted, or after a certain amount of time of inactivity. Thus, the determination can work adequately, even if there is a change of user between two sessions. There may, additionally or alternatively, be provided an option in the user interface 204 of the wearable device 201 to reset the predefined direction range. The predefined direction range may accordingly be reset if a substantial change is made in the swing movement of the user, for example if a coach tells the user to swing differently or adapt their movement in some way.

In embodiments, there is a possibility to store one or more user characteristics in the wearable device 201, and there may be provided an option in the user interface 204 of the wearable device 201 to change between two or more users and their stored respective predefined direction ranges.

The predefined direction range may comprise two or more pre-defined sub-areas. The sub-areas may be connected or separate.

Fig. 14 shows an example predefined direction range comprising a first sub-area 1102a and a second sub-area 1102b. The first sub-area 1102a may for example correspond to a golf stance position when wearable device 201 is worn on the right forearm of the user, and the second sub-area 1102b may for example correspond to a golf stance position when wearable device 201 is worn on the left forearm of the user. Because a wearable de-vice 201 worn on the left forearm will, in a golf stance position, be pointing in a different direction than a wearable device 201 worn on the right forearm, two separate sub-areas can be provided.

Similarly, a wearable device 201 worn on an underside of the forearm will, in a golf stance position, point in a different direction to a wearable device worn on an overside of the forearm. There may accordingly be provided two different sub-areas, each corresponding to a side of the forearm on which the wearable device 201 is worn.

In embodiments there may be a plurality of separate sub-areas corresponding to arm mount positions of the wearable device 201. The arm mount position may be defined as the arm and/or location on said arm where the wearable device 201 is being worn. The arm mount position may be the left arm or the right arm. The arm mount position may further be the overside or underside of the forearm. For example, if the user wears the wearable device 201 on an overside of the right forearm, then the arm mount position can be defined as being the overside of the right forearm. In embodiments the predefined direction range may comprise four sub-areas corresponding to four arm mount positions: (i) underside of left forearm, (ii) overside of left forearm, (iii) underside of right forearm, and (iv) overside of right forearm.

The inventors have realized that it is possible to configure the wearable device 201 to determine the arm mount position of the wearable device 201. This may be achieved by comparing the orientation of the wearable device 201 with the direction of gravity when the user is in a golf stance position. Depending on within which sub-area of the predefined direction range the relative direction of gravity is located, the arm mount position can be determined. For example, if the relative direction of gravity is within the sub-range corresponding to the arm mount position of the wearable device 201 being worn on the underside of the left forearm, the wearable device 201 can determine that the wearable device 201 is worn on the underside of the left forearm.

The determination of the arm mount position is beneficial for the formulation of a reference motion track of a golf swing. For example, the "ideal" reference motion track may differ depending on the arm mount position. In other words, the ideal motion track for a de-vice worn on the left arm is different from the ideal motion track for a device worn on the right arm etc. Thus, if the arm mount position is known, the reference motion track may be formulated in dependence on the determined arm mount position. Alternatively, or additionally, a reference plane (swing plane) may be formulated in dependence on the determined arm mount position. Thus, a golf swing flaw may be more accurately determined if the arm mount position is known.

The determination of the arm mount position also makes post-processing and analysis of the golf swing more accurate. For example, the tracked motion captured by the wearable device will differ depending on the arm mount position, i.e. the motion track from a wearable device worn on a left forearm will be different to a motion track from a wearable device worn on the right forearm, even if the golf swings are identical. Thus, by knowing the arm mount position, this difference can be compensated for during analysis and post-processing of the tracked golf swing. Higher quality analysis can thus be performed, and more accurate golf swing characteristics may be obtained.

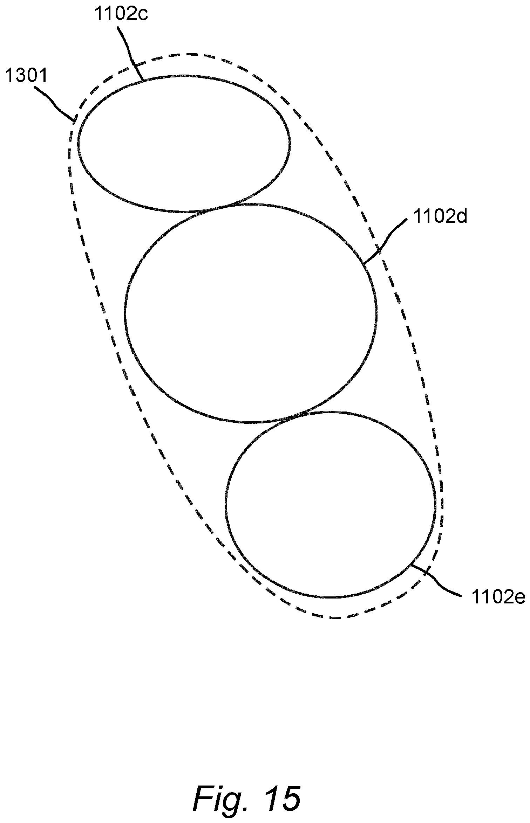

Fig. 15 shows an exemplary predefined direction range (in 2D) comprising a plurality of sub-areas 1102c, 1102d and 1102e. The plurality of sub-areas 1102c, 1102d, and 1102e may each correspond to a different golf club or golf club group. Club groups may include drivers, woods, irons, wedges, or the like, while clubs may include several specific clubs per club group, e.g., irons may include 9 iron, 8 iron, 7 iron, 6 iron, and so on. For example, sub-area 1102c may correspond to a first golf club, sub-area 1102d may correspond to a second golf club and so on. Similarly, sub-area 1102c may correspond to a first golf club group, sub-area 1102d may correspond to a second golf club group and so on. Although three sub-areas are shown in Fig. 15, it will be appreciated that the predefined direction range may comprise fewer, or more, sub-areas. It will also be appreciated that the sub-areas shown are purely illustrative and that their size and shape will differ depending on user, club, and club group.

In the simplified example shown in Fig. 15, sub-area 1102c corresponds to a driver, sub-area 1102d corresponds to iron clubs, and area 1102e corresponds to wedges.

As clubs and club groups tend to be of different length, the golf stance position will differ, for a specific user, when using different golf clubs or golf club groups. This allows the sub-areas corresponding to the different golf clubs or golf club groups to be distinct. For example, a user performing a golf swing with a driver will hold the club at a shallower angle (i.e. standing further away from the ball) than the same user performing a golf swing with a wedge. The position of the ball, in a forward-backward direction, will also differ between the clubs.

The inventors have realized that it is possible to configure the wearable device 201 to determine with which golf club or golf club group the user is performing a golf swing. This may be achieved by comparing the orientation of the wearable device 201 with the direction of gravity when the user is in a golf stance position. Depending on within which sub-area of the predefined direction range the relative direction of gravity is located, the club or club group can be determined. For example, if the relative direction of gravity is within the sub-range corresponding to a first golf club or golf club group, the wearable device 201 can determine that golf swing is being performed with a first golf club or golf club group respectively. As another example, if the relative direction of gravity is within the sub-area corresponding to the club being a driver, the wearable device 201 can determine that the user is using a driver as their golf club. In an embodiment, the wearable device 201 is capable of distinguishing between two or more club groups. In an embodiment, the wearable device 201 is capable of distinguishing between two or more clubs.

The determination of which club, or club group, the user is using, is beneficial for the formulation of a reference motion track of a golf swing. For example, the "ideal" reference motion track may differ depending on the club or club type. In other words, the ideal motion track for a golf swing with one club (or club group) may be different from the ideal motion track for a golf swing with another club (or club group). Thus, if the club or club group is known, the reference motion track may be formulated in dependence on the determined club or club group. Alternatively, or additionally, a reference plane (swing plane) may be formulated in dependence on the determined club or club group. Thus, a golf swing flaw may be more accurately determined if the club or club group is known.

The determination of which club, or club group, the user is using, also makes post-processing and analysis of the golf swing more accurate. For example, an ideal golf swing with a driver may differ from an ideal golf swing with an iron club and/or a wedge. Thus, by obtaining information about the golf club, from the golf stance position, the wearable de-vice 201 can perform higher quality analysis that is tailored to the specific club and/or club group.

It should be noted that both the determination of arm mount position and the determination of golf club or golf club group can be made both in conjunction with the golf stance detection and independently of the golf stance detection. It will be appreciated that it is not necessary for the wearable device 201 to detect the golf stance position (e.g. according to the methods of Fig. 8 or 10) in order to determine the arm mount position or the golf club or golf club group. The determination of arm mount position or the golf club or golf club group may instead be performed during post-processing of the tracked motion. The wearable device 201 may, for example, determine during post-processing that the golf stance position is the position taken before the golf swing is performed, and compare the relative direction of gravity at that point of the tracked motion to the predefined direction range and its sub-areas.

The wearable device 201 may further be configured to provide information about the tracked golf swing to a user. The information may be one or more golf swing characteristics, such as tempo, the length of backswing (LoB), maximum hand speed, transition, or other characteristics indicative of the quality of a golf swing.

The wearable device 201 may provide the information to the user via the user interface 204 (e.g. via the indicator 208 or a display) or by transmitting the information to a remotely located system, such as a computer, mobile phone, smartphone or the like. The remotely located system may display one or more golf swing characteristics to the user. This allows the user to see what their golf swing looks like, and to learn what adjustments are needed.

Although specific reference has been made to the use of embodiments in the context of golf swings, it is appreciated that where the context allows, embodiments may include tracking of other sports motions such as baseball bat strokes, pole vaults, discus throws or other repetitive sports motions.

Throughout this specification, the word "may" is used in a permissive sense (i.e. meaning having the potential to), rather than in the mandatory sense (i.e. meaning must).

Throughout this specification, the words "comprise", "include", and variations of the words, such as "comprising", "comprises", "including" and "includes", do not exclude other elements or steps.

As used throughout this specification, the singular forms "a", "an", and "the", include plural referents unless explicitly indicated otherwise. Thus, for example, reference to "an" element includes a combination of two or more elements, notwithstanding use of other terms and phrases for one or more elements, such as "one or more" or "at least one".

The term "or" is, unless indicated otherwise, non-exclusive, i.e. encompassing both "and" and "or". For example, the feature "A or B" includes feature "A", feature "B" and feature "A and B".

Unless otherwise indicated, statements that one value or action is "based on" and/or "in dependence on" another condition or value or action, encompass both instances in which the condition or value or action is the sole factor and instances where the condition or value or action is one factor among a plurality of factors.

Unless otherwise indicated, statements that "each" instance of some collection have some property should not be read to exclude cases where some otherwise identical or similar members of a larger collection do not have the property, i.e. each does not necessarily mean each and every.

## Claims

1. A wearable device for improving a user's golf swing, the wearable device comprising:
a sensor unit configured to sense motion sensor data relating to acceleration and rotation of the wearable device in three-dimensional space;
a stimulator for providing stimuli to the user of the wearable device;
a processor;
a central housing for housing the sensor unit and the stimulator; and
an attachment member for attaching the central housing to a forearm of the user;
wherein the processor is configured to:
determine, in dependence on the motion sensor data, a relative direction of gravity relative to an orientation of the wearable device; and
detect that the user is in a golf stance position in dependence on the determined relative direction of gravity being within a pre-defined direction range indicative of a golf stance;
wherein, in response to the detection of a golf stance position, the processor is further configured to:
determine a motion track of the wearable device in dependence on motion sensor data from the sensor unit; and
transmit a stimuli signal to the stimulator causing the stimulator to provide stimuli to the user at a time when a golf swing flaw is detected in the determined motion track.

2. The wearable device according to claim 1, wherein the detection of the golf stance position is made in dependence on the determined relative direction of gravity being within the pre-defined direction range for an amount of time longer than a threshold amount of time.

3. The wearable device according to claim 1 or 2, wherein the processor is configured to determine if the wearable device is stationary, and wherein the detection of the golf stance position is made in dependence on the wearable device being determined to be stationary.

4. The wearable device according to any of claims 1 to 3, wherein the processor is configured to adapt the predefined direction range in dependence on a plurality of previously tracked golf swings, preferably in dependence on three to ten previously tracked golf swings, more preferably in dependence on three to five previously tracked golf swings.

5. The wearable device according to claim 4, wherein the predefined direction range is adapted in dependence of the determined relative direction of gravity for each of the plurality of previously tracked golf swings.

6. The wearable device according to claim 5, wherein the predefined direction range is adapted in dependence on either:
an average and variation amount of the determined relative direction of gravity of each of the plurality of tracked golf swings; or
an outlining of the determined relative direction of gravity of each of the plurality of tracked golf swings.

7. The wearable device according to any of claims 4 to 6, wherein the processor is further configured to determine if a golf swing has been completed, and wherein the plurality of previously tracked golf swings are golf swings that have been determined to be completed.

8. The wearable device according to any preceding claim, wherein the wearable device further comprises an indicator for indicating an internal state of the wearable device or a state of the tracked motion, wherein the indicator is configured to indicate to the user in dependence on detection of a golf stance position.

9. The wearable device according to any preceding claim, wherein the attachment member is one of a strap for a bracelet, a sleeve, or a glove.

10. The wearable device according to any preceding claim, wherein:
an arm-facing surface of the central housing or the attachment member comprises, or is provided with, a friction enhancing material;
an arm-facing surface of the central housing comprises a curved portion; or
the attachment member comprises a tensioning means for tensioning the attachment member to the forearm of the user.

11. The wearable device according to any preceding claim, wherein the processor is further configured to:
determine, in dependence on the motion sensor data, a relative direction of gravity relative to the orientation of the wearable device when the user is in a golf stance position;
determine one or more of (a) an arm mount position of the wearable device, (b) a golf club used by the user, and (c) a golf club group used by the user, by comparing the determined relative direction of gravity to a plurality of sub-ranges of a pre-defined direction range, each sub-range being indicative of (a) an arm mount position of the wearable device, (b) a golf club used by the user, or (c) a golf club group used by the user.

12. A method of detecting a golf stance position of a user, the method comprising:
determining, in dependence on motion sensor data from a wearable device worn on a forearm of the user, a relative direction of gravity relative to an orientation of the wearable device; and
detecting that the user is in the golf stance position in dependence on the determined relative direction of gravity being within a pre-defined direction range indicative of a golf stance.

13. A method of improving a golf swing of a user, the method comprising:
determining, in dependence on motion sensor data from a wearable device worn on a forearm of the user, a relative direction of gravity relative to an orientation of the wearable device;
detecting that the user is in a golf stance position in dependence on the determined relative direction of gravity being within a pre-defined direction range indicative of a golf stance;
determining, in response to the detection of the golf stance position, a motion track of the wearable device in dependence on motion sensor data from the wearable device; and
transmitting a stimuli signal to a stimulator of the wearable device causing the stimulator to provide stimuli to the user at a time when a golf swing flaw is detected in the determined motion track.

14. A wearable device for improving a user's golf swing, the wearable device comprising:
a sensor unit configured to sense motion sensor data relating to acceleration and rotation of the wearable device in three-dimensional space;
a stimulator for providing stimuli to the user of the wearable device;
a processor;
a communication unit for communicating with a remotely located device;
a central housing for housing the sensor unit, the stimulator, and the processor; and
an attachment member for attaching the wearable device to a forearm of the user;
wherein the processor is configured to:
determine, in dependence on the motion sensor data, a relative direction of gravity relative to an orientation of the wearable device when the user is in a golf stance position;
determine one or more of (a) an arm mount position of the wearable device, (b) a golf club used by the user, and (c) a golf club group used by the user, by comparing the determined relative direction of gravity to a plurality of sub-ranges of a pre-defined direction range, each sub-range being indicative of (a) an arm mount position of the wearable device, (b) a golf club used by the user, or (c) a golf club group used by the user;
determine a motion track of the wearable device in dependence on motion sensor data from the sensor unit; and
transmit a stimuli signal to the stimulator causing the stimulator to provide stimuli to the user at a time when a golf swing flaw is detected in the determined motion track.
